# EUROPEAN PATENT SPECIFICATION

(11) **EP 1 410 790 B1**
(45) Date of publication and mention of the grant of the patent: **23.06.2010**
(21) Application number: 03103720.3
(22) Date of filing: 07.10.2003
(51) Int. Cl.: A61K 9/127, A61K 31/19

(54) **Submicron-liposome containing triterpenoid and a method for preparing the same**
Triterpenoid-haltige Submicronliposomen und ihre Herstellungsweise
Submicro-Liposomes comprenant des triterpenoides et la méthode de préparation

(30) Priority: 09.10.2002 KR 2002061448
(43) Date of publication of application: 21.04.2004
(73) Proprietor: Amorepacific Corporation, Seoul (KR)
(72) Inventor: Kang, Hyung-seok, Kyunggi-do (KR); Nam, Gae-Won, Seoul (KR); Han, Sang-Hoon No.2-203, Chullok Appartment 276-3, Kyunggi-do (KR); Chang, Ih-Seop No.102-1104, Dongbo Appartment 703, Kyunggi-do (KR)
(74) Representative: Poulin, Gérard

(56) References cited:
- WO-A-01/17523
- DATABASE WPI Section Ch, Week 200371 Derwent Publications Ltd., London, GB; Class A96, AN 2003-753305 XP002267648 & KR 2003 026 414 A (ENPRANI CO LTD), 3 April 2003 (2003-04-03)
- BOTH DAWN M ET AL: "Liposome-encapsulated ursolic acid increases ceramides and collagen in human skin cells" ARCHIVES OF DERMATOLOGICAL RESEARCH, vol. 293, no. 11, January 2002 (2002-01), pages 569-575, XP002265521 ISSN: 0340-3696
- SUK K H ET AL: "OLEANOLIC ACID AND URSOLIC ACID STABILIZE LIPOSOMAL MEMBRANES" LIPIDS, CHAMPAIGN, IL, US, vol. 32, no. 7, 1997, pages 769-773, XP000929341 ISSN: 0024-4201
- SON LE BANG ET AL: "Liposomal form of betulinic acid, a selective apoptosis inducing in melanoma cells substance" JOURNAL OF LIPOSOME RESEARCH, vol. 8, no. 1, February 1998 (1998-02), page 78 XP009023312 Sixth Liposome Research Days Conference;Les Embiez, France; May 28-31, 1998 ISSN: 0898-2104

## Description

### FIELD OF THE INVENTION

The present invention relates to a liposome containing triterpenoid and a method for preparing the liposome. More particularly, the present invention relates to a submiron-liposome obtained by capturing water- or solvent-insoluble triterpenoid into a bilayer liposome at a high concentration and to a method for preparing the submiron-liposome.

### BACKGROUND OF THE INVENTION

Liposome is a colloid particle of aqueous dispersion type and formed by a self-assembling process of lipid molecules that have inner aqueous phase separated by hydrophobic phospholipid bilayer. Liposome was first introduced in 1965 for artificial cell membrane research (J. Mol. Biol. 13: 238-252), and has been widely studied for drug delivery carriers since 1970's. It has been applied to various uses based on its colloid and surface property, drug carrying ability, and amphiphilic property. For examples, it has been applied for pharmaceutical applications including delivery carriers of antibacterial, anticancer drug and vaccine development and for cosmetic applications including skin care products and hair conditioning products.

However, the early-made liposome developed for drug delivery system was not fully satisfactory due to its biological and colloidal instability and irregular amount of encapsulation. Thus, methods for increasing the stability of liposome were extensively studied, and as a result, the stability was improved by introducing polymeric stabilizer and biological molecules stabilizing liposome bilayer. Since then, liposome-based products such as antibacterial or anticancer substances have been invented.

Liposome, due to its vesicular properties, can solubilize hydrophobic compounds into its hydrophobic membrane bilayer and can encapsulate hydrophilic compounds in its aqueous core. Because of these properties, liposome has been applied to encapsulation carrier for many substances. Liposome can encapsulate such compounds as having low solubility or high toxicity that is not allowable for medical dosage, therefore, it is very useful for drug carrier, which can reduce the toxicity and deliver bioactive substances for a long time.

There are two different kinds of methods to prepare submicron-liposome.

In the present invention, submicron-liposome means nano-scaled liposome.

The first method is inducing spontaneous formation of liposome by the self-assembly of the lipid molecules. This method includes, for examples, a spontaneous formation of vesicles by controlling pH, by adding surfactant, or by removing surfactant from mixed micelle system.

The method of controlling pH is limited to specific phospholipids whose solubility is changed according to pH. In this method, the difference of pH between the inside and the outside of the liposome results in different curvature of inside and outside of the liposome, which leads to spontaneous formation of the submicron-liposome. However, this method cannot be applied to the phosphatidylcholine-based lipids that are most generally used in the preparation of the liposome.

The method of adding surfactant uses single-chained surfactant in order to correct curvature of the phosphatidylcholine to that of a circle. Adding a surfactant results in a smaller liposome compared with that of no addition, but the liposome is still lager than those of the former methods.

The method of removing surfactant from mixed micelle system is achieved by gradually removing ionic surfactant from micelle system comprising ionic surfactant and phospholipid. This method results in a minute and uniform liposome, but it is not applicable to mass production, and needs special equipment to remove surfactant and very long manufacturing time.

The second method uses mechanical force or energy such as extrusion, french press, high pressurizing, ultrasonication treatments or the like to micronize hydrated or swollen liposome. The procedure of this method can be divided in two steps; the first step is preparing large multilamellar liposome and the second step is micronizing the large multilamellar liposome.

In the first step, lipid is dissolved in a volatile organic solvent and dried under a reduced pressure to remove solvent so as to form a lipid film on the walls of the container; and then, aqueous dispersant is added thereto to hydrate and swell out the lipid film to form a liposome. Because the above-obtained liposome is multilamellar vesicle type and has large diameters in a range of several-several hundreds of micrometer, the large vesicle is subjected to a mechanical force or energy of the second step to obtain smaller liposome. Each of the second steps is different only in the form or way of applying energy to micronize the liposome.

Submicron-liposome also can be obtained by dissolving a lipid in an ethanol to prepare an ethanol solution of lipid and then adding the ethanol solution into an aqueous dispersant gradually. Since the liposome property prepared by this method depends on the addition rate of the ethanol solution and lipid concentration in ethanol, it is difficult to form highly concentrated liposome. In addition, this method needs additional process for removing a large quantity of ethanol used. (D.D Lasic, Liposomes: From physics to applications, 1993, Elsevier science B.V.).

WO 01/17523 describes a ursolic acid-containing liposome made by dissolving ursolic acid in an ethanol solution comprising phosphatidyl choline and cholesterol.

Thus, it has been desired a new method for preparing submicron-liposome without applying additional compound or mechanical force.

Triterpenoid is a natural bioactive compound and is a general name of a pentacyclic compound including ursolic acid, betulinic acid, boswellic acid, oleanolic acid, etc. It has been reported that triterpenoid has effects on anti-cancer, hepatic protection, anti-inflammation, anti-ulcerative, antibacterial, anti-scorbutic, antiviral, collagen biosynthesis, lipid biosynthesis, or the like. However, triterpenoid is hardly insoluble in both water and solvent, resulting in that it is difficult to be incorporated into cosmetics more than 0.02% by weight. (Rios, J.L., Recio, M.C., Manez, S., Giner, R.M., 2000. Natural triterpenoids as anti-inflammatory agents. In: Atta-Ur-Rahman Ed., Studies in Natural Products Chemistry: Bioactive Natural Products, vol. 22. Elsevier science, Amsterdam, pp. 93-143, part C.)

It has been reported that when a hydrophilic or lipophilic compound is loaded in a liposome, skin penetration of the compound is improved and therefore, the biological efficacy of the compound can be increased. Because of this reason, the studies on the liposome containing triterpenoid at high concentration have been extensively conducted, but the results of these studies are not satisfactory because the content of triterpenoid in an aqueous solution is still too small and toxic solvent is needed. (Both, D.M., Goodtzova, K., Yarosh, D.B., Brown, D.A., 2002. Liposome-encapsulated ursolic acid increases ceramides and collagen in human skin cells. Arch. Dermatol. Res. 293, 569-575; Ostro, M.J., Liposomes: From Biophysics to Therapeutics, Marcel Decker, New York, 1987.) Therefore, in order to apply the triterpenoid to the cosmetics, it is necessary to find a method for encapsulating the triterpenoid at high concentration while using non-toxic solvent.

Under these circumstances, in order to overcome the above-described technical limitations of applying triterpenoid, the present inventors have done extensive studies on the preparation of a triterpenoid liposome and the chemical properties of triterpenoid. As a result, the inventors have succeeded in obtaining submicron-liposome having triterpenoid uniformly loaded therein and certifying their activities.

### SUMMARY OF THE INVENTION

The present invention provides a method for preparing submicron-liposome containing triterpenoid at high concentration while using non-toxic solvent without intense mechanical treatment.

The present invention also provides submicron-liposome containing triterpenoid at high concentration.

Further, the present invention provides cosmetic compositions containing bioactive triterpenoid liposome as described above.

### BRIEF DESCRIPTION OF THE DRAWINGS

Fig. 1 is the picture of transmission electron microscope synthesized by the liposome of Example 5.
Fig. 2 is the result of wide-angle x-ray diffraction by the liposomes of Examples 1, 3, 4, 5, and 6 and oleanolic acid (OA).
Fig. 3 is the intermolecular spacing of lecithin molecules by the liposomes of Examples 3, 4, 5, and 6.
Fig. 4a is the result of thin layer liquid chromatography of ceramide synthesized by the liposome of Example 1.
Fig. 4b is the result of thin layer liquid chromatography of ceramide synthesized by the liposome of Example 5.
Fig. 5 is the result of western blotting of proteins expressed by the liposomes of Example 1 and 5.

### DETAILED DESCRIPTION OF THE INVENTION

The following is a detailed description of the present invention.

The present invention provides a method for preparing submicron-liposome containing triterpenoid at high concentration, wherein the triterpenoid is uniformly loaded and can exhibit biological activity.

In the present invention, submicron-liposome means micronized liposome.

Specifically, in order to incorporate triterpenoid at a high concentration and uniformly into a liposome, the present invention employs triterpenoid having acid group, and by adding a base, the triterpenoid is transformed into its salt with surface activity. The transformed triterpenoid salt is a surfactant of high HLB, and it forms mixed micelle system when mixed with low HLB lipid. The above-obtained mixed micelle system has minute diameter of about 1~100 nm and maintains its pH in a range of 10-11. In addition, by adding an acid to decrease its pH to 5~8, the triterpenoid salt transforms back to the original form having acid group, and thereby loses its surface activity resulting in changing the mixed micelle system into a liposome. During the transformation, triterpenoid is loaded into the liposome at high concentration.

A method for preparing the present submicron-liposome containing triterpenoid at high concentration may comprise the following steps of:
(a) dispersing triterpenoid into a polyol while heating up to 60~70°C;
(b) adding a base into the dispersion of step (a) to decrease its viscosity;
(c) dissolving phospholipid in an ethanol at room temperature;
(d) adding the ethanol solution of step (c) into the dispersion of step (b);
(e) adding the mixture of step (d) into distilled water and then emulsifying; and
(f) adding an acid to the emulsion of step (e) to prepare submicron- liposome.

Hereinafter, the submicron-liposome may be called as "triterpenoid liposome.

In step (e) and (f) the mixture is micelle type, and the micellar mixture is transformed into liposome in step (f) while returning the triterpenoid salt to an acid type.

In the liposome obtained by the above method, triterpenoid is uniformly contained in liposomal bilayer at high concentration.

The present invention is described hereinafter in more detail.

In the present invention, lecithin may be preferably employed as a phospholipid.

Lecithin employed in the present invention is conventionally extracted from soybeans or yolks and refined. Lecithin is a phospholipid with fatty acid chain of 12-24 carbons, comprising phosphatidylcholine, phosphatidylethanolamine, phosphatidylserine, phosphatidylglycerol, phosphatidylinositol, phosphatidic acid and other fatty acids obtained by hydrolysis thereof.

It is preferably to employ an unsaturated lecithin with hydrophobic group having about 0~3 of double bonds, and a mixture of lecithin containing 70~95 wt% of phosphatidylcholine depending on the purification is preferable. And, because the double bonds of fatty acid chain are easily oxidized by water or active oxygen, partially hydrogenated lecithin may also be employed in order to improve chemical stability. More preferably, unsaturated lecithin containing 90~95 wt% of phosphatidylcholine isolated from soybeans may be employed, and it may be employed in an amount of 0.001~15% by weight, and preferably, 1~10% by weight based on the total weight of the liposome.

Active components loaded into the liposome of the present invention are triterpenoids having one or more acid group, for example, ursolic acid, oleanolic acid, centella asiatica extract, betulinic acid and β-boswellic acid are useful triterpenoids. The content of triterpenoid in the present triterpenoid liposome may be in a range of 0.001~5% by weight, and preferably, 0.5~2.5% by weight based on the total weight of liposome.

In order to load the triterpenoid into liposome, triperpenoid is dispersed in a polyol while heating, and cooled to a room temperature, and then a base is added to prepare dispersion with low viscosity. Ethanol solution of lecithin prepared by dissolving a lecithin in ethanol is added to the dispersion, and then dissolved at a room temperature entirely. The above-obtained mixture is added into distilled water and then emulsified to prepare translucent dispersion system.

A base employed at this step may be triethanolamine, triisopropanolamine, potassium hydroxide, 2-aminobutanol, sodium hydroxide, ammonium hydroxide, calcium hydroxide, or the like. Preferably, a base with same normality as that of the triterpenoid may be added in an amount of 0.001~0.5% by weight based on the total weight of liposome to control the pH as 10-11.

Finally, by adding an acid to the translucent dispersion system, the translucent dispersion system is transformed to liposome dispersion. Preferably, pH of the final liposome dispersion is controlled to be in a range of pH 5~8. In order to control the pH, an acid with the same normality as that of the above base is added to the translucent dispersion system, which leads to transformation from mixed micelle to liposome. The acid comprises adipic acid, boric acid, citric acid, acetic aid, formic acid, fumaric acid, lactic acid, glycolic acid, succinic acid, propionic acid, pyruvic acid, phosphoric acid, or the like. As the result of the addition of acid, the submicron-liposome of the present invention containing triterpenoid is finally obtained.

Triterpenoid liposome provided by the present invention may have a diameter in a range of 0.001~10 µm, preferably 0.1~1.0 µm, and more preferably 0.1~0.2 µm. The size of the triterpenoid liposome is not restricted, and it is preferable to contain uniform-sized liposome. In this case, the size of the uniform-sized liposome may be preferably 0.1~1.0 µm.

For the stability of the liposome dispersion, a polymer thickner may be employed, and the polymer thickner may comprise polyacrylic acid; polymethacrylic acid; polyurethane having polyethylene glycol as a hydrophilic group; polyvinyl alcohol; di-or tri-block copolymer of polyethyleneoxide and polypropylene oxide; xanthan gum; hyaluronic acid; hydroxyethylcellulose; hydroxypropylmethylcellulose; carboxymethylcellulose; hydroxybutylmethyl- cellulose; methyl hydroxyethylcellulose; polyglutamic acid; chitosan, etc, and may be employed in an amount of 0.1~0.5% by weight.

The preparation method and the triterpenoid submicron-liposome of the present invention have the following advantages.

First, triterpenoid can be loaded into the submicron-liposome at a high concentration while using non-toxic solvent;

Second, because insoluble triterpenoid is loaded in a liposome at a high concentration, it can exhibit much higher biological activity than conventional triterpenoid existing as a free crystal state;

Third, skin penetration of triterpenoid can be promoted by the improved skin absorption of the unsaturated lecithin (phospholipid).

Fourth, because the transition from a dispersion system to a liposome is self-assembling process, mechanical treatment is not required and thereby submicron-liposome having uniform size can be achieved;

The submicron- liposome containing triterpenoid may be incorporated into skin-care compositions including cosmetic compositions, pharmaceutical or quasi-pharmaceutical compositions, and its formulation is not limited. For examples, it may be formulated, but not limited thereto, into basic skin-care products, make-up products, cleansing products, hair-styling products, hair-tonic, hairdyes, lotion, cream, gel, patch, sprays, or the like.

For example, the skin-care composition of the present invention may have a formulation of skin softener, toilet water, nutrition toilet water, nutrition cream, massage cream, essence, eye cream, eye essence, cleansing cream, cleansing foam, cleansing water, pack, powder, body lotion, body oil, body essence, make-up base, foundation, hairdyes, shampoo, body cleaner, tooth paste, oral cleaner, patch, sprays, or the like

### PREFERRED EMBODIMENT OF THE INVENTION

The present invention will be described in more detail by the following examples and experimental examples, which should not be considered to restrict the scope of the present invention.

### <Example 1>

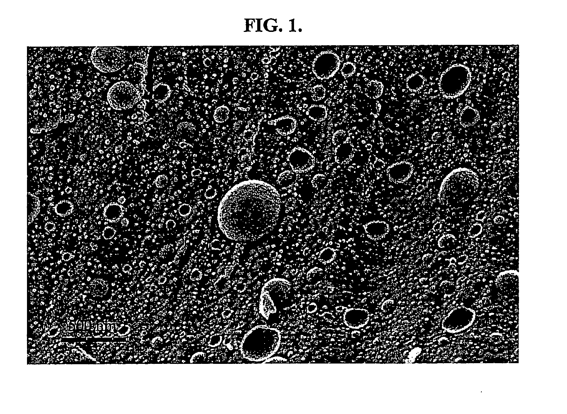

2 g of ursolic acid (UA) was dispersed in 15 g of butylene glycol while heating. 1.4 g of potassium hydroxide was added to the above-obtained dispersion and dissolved entirely. 10 g of unsaturated lecithin containing 90~95 wt% of phosphatidylcholine isolated from soybeans was dissolved in 4g of ethanol and then mixed with the above dispersion to obtain transparent solution. This solution was added to 64 g of distilled water and then agitated for 30 minutes at 300 rpm, at a room temperature. 1.8 g of citric acid was added thereto to obtain the aimed liposome containing 2 wt% of ursolic acid. Prepared liposomes are visualized by transmission electron microscope in Fig. 1.

### <Example 2>

2 g of betulinic acid was dispersed in 15 g of butylene glycol while heating. 1.7 g of potassium hydroxide was added to the above-obtained dispersion, and dissolved entirely. 10 g of unsaturated lecithin containing 90-95 wt% of phosphatidylcholine isolated from soybeans was dissolved in 4 g of ethanol and then mixed with the above dispersion to obtain transparent solution. This solution was added to 64 g of distilled water and then agitated for 30 minutes at 300 rpm, at a room temperature. 1.9 g of citric acid was added thereto to obtain the aimed liposome containing 2 wt% of betulinic acid.

### <Comparative Example 1 and Examples 3, 4, 5 and 6>

Oleanolic acid as shown in Table 1 was dispersed in 5 g of propylene glycol and 10g of ethanol while heating. Potassium hydroxide as shown in Table 1 was added to the above-obtained dispersion and dissolved entirely. 10 g of unsaturated lecithin containing 90~95 wt% of phosphatidylcholine isolated from soybeans was dissolved in 4 g of ethanol and then mixed with the above dispersion to obtain transparent solution. This solution was added to 64 g of distilled water and then agitated for 30 minutes at 300 rpm, at a room temperature. Citric acid as shown in Table 1 was added thereto to obtain the aimed liposome containing oleanolic acid as shown in Table 1. In Comparative Example 1, no oleanolic acid, no potassium hydroxide no citric acid were added.

**[Table 1]**

| The contents of oleanolic acid, potassium hydroxide and citric acid | | | | | |
|---|---|---|---|---|---|
| | C. Ex. 1 | Ex. 3 | Ex. 4 | Ex. 5 | Ex. 6 |
| Oleanolic acid (g) | 0 | 0.5 | 1 | 2.0 | 2.5 |
| Potassium hydroxide (g) | 0 | 0.35 | 0.7 | 1.4 | 1.75 |
| Citric acid (g) | 0 | 0.44 | 0.88 | 1.75 | 2.18 |
| Content of oleanolic acid in liposome (wt%) | 0 | 0.5 | 1.0 | 2.0 | 2.5 |

### <Example 7>

1 g of ursolic acid, 0.7 g of oleanolic acid and 0.5 g of centella asiatica extract were dispersed in a mixed-solvent of 13 g of butylene glycol and 2.5 g of propylene glycol while heating. 1.2 g of potassium hydroxide was added to the above-obtained dispersion, and dissolved entirely. 10 g of unsaturated lecithin containing 90~95 wt% of phosphatidylcholine isolated from soybeans was dissolved in 4 g of ethanol and then mixed with the above dispersion to obtain transparent solution. This solution was added to 64 g of distilled water and then agitated for 30 minutes at 300 rpm, at a room temperature. 1.48 g of citric acid was added thereto to obtain the aimed liposome containing 1.0 wt% of ursolic acid, 0.7 wt% of oleanolic acid and 0.5 wt% of centella asiatica extract.

### <Experimental Example 1> Particle size measurement by using dynamic light scattering particle size distribution analyzer

Average particle size of triterpenoid liposome prepared in Comparative Example 1 and Examples 1-7 was measured by using dynamic light scattering particle size distribution analyzer. The scattering angle was fixed as 90° and temperature was maintained as 25°C while measuring. The results are shown in Table 2.

**[Table 2]**

| Average size and stability of liposome prepared in Examples and C. Example | | | | | | | | |
|---|---|---|---|---|---|---|---|---|
| | C. Ex. 1 | Ex. 1 | Ex. 2 | Ex. 3 | Ex. 4 | Ex. 5 | Ex.6 | Ex. 7 |
| Average particle size(µm) | 0.879 | 0.175 | 0.253 | 0.533 | 0.225 | 0.166 | 0.278 | 0.129 |
| Degree of precipitation | 3 | 1 | 1 | 2 | 1 | 1 | 2 | 1 |

| | | | | | | | | |
|---|---|---|---|---|---|---|---|---|
| [Note] Degree of precipitation: ranges 1~5, higher number means greater quantity of precipitate | | | | | | | | |

The liposome prepared in the present invention has the diameter within the range of 0.1∼0.3 µm, which is very uniform and smaller than that of liposome prepared in Comparative Example 1. In addition, the present liposome is very uniform in its size and has sub-micron type having very low precipitation intensity, which means a good colloidal stability. Compared with the Comparative Example 1, all the liposome of the present invention containing triterpenoid was self-assembled sub-micron liposome, regardless of the kinds and content of triterpenoid.

### <Experimental Example 2> Wide angle X-ray diffraction

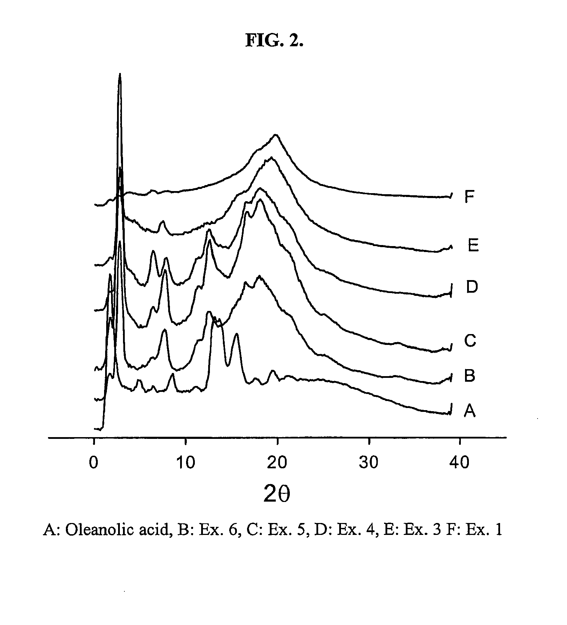

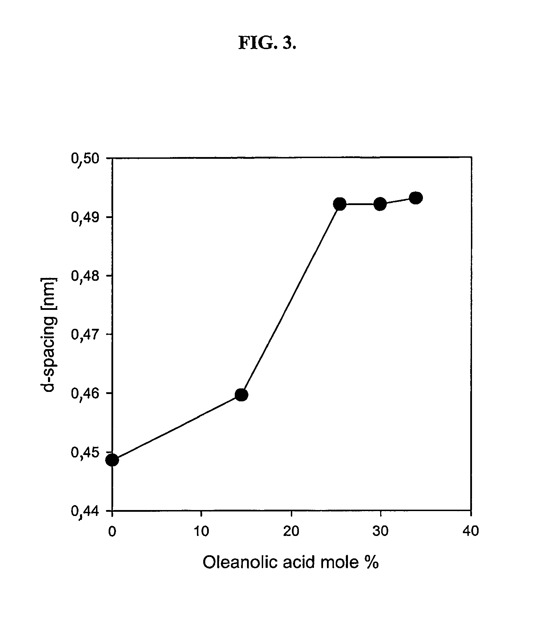

The structures and the crystallinities (crystalline property) of the liposome were analyzed with powder XRD equipped with wide-angle diffractometer (Rigaku/USA, D/max-RB) using Cu-Kα (λ=1.54 Å) ray. The oleanolic acid (OA) and liposome prepared in Examples 1, 3, 4, 5 and 6 and frozen-dried were analyzed by X-ray diffraction in wide-angle (θ = 2-50°), and the structures of hydrocarbon chains and the molecular distances of oleanolic acid (OA) crystals were determined by the result of the X-ray diffraction. As can be seen in Fig. 2, specific peak of OA crystal was not observed in the liposome encapsulating the OA. Diffractions in a wide range of near 20° were observed because lipids formed smectic (lamellar) liquid-crystal phase, which correspond to the distances between lipid molecules. As can be seen in Figs. 2 and 3, 2θ value at maximum diffraction (peak) increased as the contents of OA increased and became saturated (no more increasing) at 25.4 mole % of OA, which is resulted from the increase of the distance between lecithin molecules (intermolecular space) due to the addition of OA into the bi-layer of the liposome, and at 25.4 mole % of OA, OA is saturated in the bi-layer and therefore OA is not able to be added any more.

### <Experimental Example 3> Ceramide biosynthesis

The quantity of ceramide synthesized in the skin of the hairless mice treated with the liposome was measured according to the kinds of triterpenoid liposome. The liposome prepared above was applied onto the back of the mice, and then, the back was cut into 8 mm² area and preserved in the refrigerator at -20°C. 2.5% of trypsin-ethylenediaminetetraacetic acid was added thereto, and then epidermis was separated from dermis. Lipid was extracted only from the epidermis and analyzed with a thin layer liquid chromatography, and then evaluated by CAMAG colorimeter.

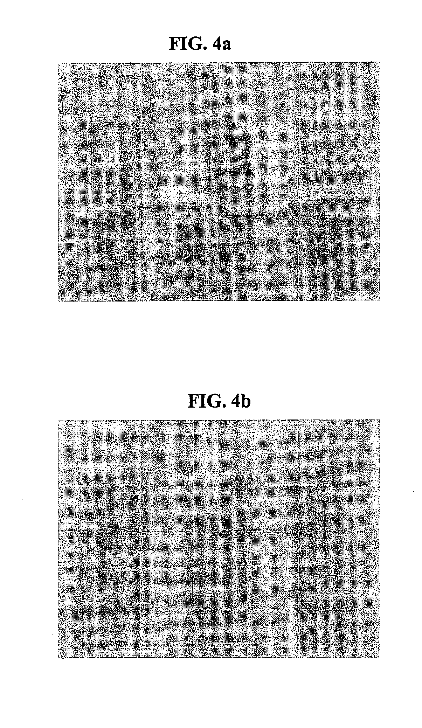

Synthesized ceramide was found from all the samples of Examples 1-6 and Comparative Example 1 and the results were illustrated in Fig. 4a and Fig. 4b. Compared with the synthesis of the ceramide when the liposome of Comparative Example 1 was treated, treatment of the liposome of Example 1 led to 180% increase in the biosynthesis of ceramide, and Examples 3, 4, 5, 6 showed 143%, 174%, 212% and 232% of increase, respectively.

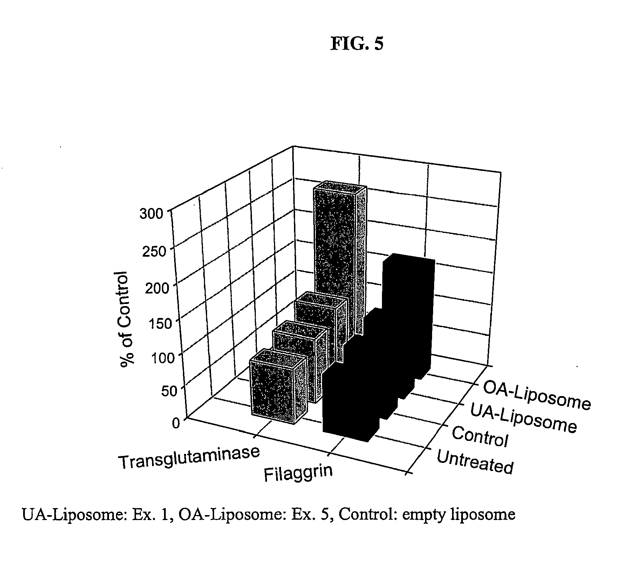

Western blotting of proteins extracted from epidermal layers of hairless mouse was carried out to reveal the degree of keratinocyte differentiation of triterpenoid-containing liposomes. Only OA-containing liposome showed distinctive protein expressions, whereas UA-containing liposome and control (empty liposome) showed the similar results to untreated sample as seen in Fig. 5. Among expressed proteins, filaggrin is a precursor of natural moisturizing factors that results from keratinocyte differentiation, and transglutaminase is a kind of differentiation markers. Both of OA- and UA-containing liposomes significantly increased the amount of total ceramides in hairless mouse skin. The Western blotting analysis was performed according to "Yarosh, D.B., Both, D., Brown, D., 2000. Liposomal ursolic acid (merotaine) increases ceramides and collagen in human skin. Hormone Res. 54, 318-321".

This result confirms that triterpenoid loaded in the present liposome exhibits more effective activities.

Thus, the present liposome can enhance ceramide biosynthesis of the skin by containing triterpenoid at high concentration to improve skin condition.

Formulations of the present invention were described hereinafter. Each of the skin application was prepared according to the tables below and conventional manufacturing methods, using liposome dispersion of the present invention.

### <Formulation 1> Skin softner

**[Table 3]**

| Materials | Composition (% by weight) |
|---|---|
| Betain | 3.0 |
| Natto gum | 3.0 |
| Cellulose gum | 0.005 |
| Ethanol | 5.0 |
| Preservative | 0.5 |
| Liposome dispersion of Example 4 | 5.0 |
| Polyoxyethylene hydrogenated castor oil | 0.2 |
| Tocopheryl acetate | 2.0 |
| Pigments | q.s. |
| Perfume | q.s. |
| Distilled water | To 100 |

### <Formulation 2> Nutrition toilet water

**[Table 4]**

| Materials | Composition (% by weight) |
|---|---|
| Cetyl ethyl hexanoate | 4.0 |
| Cetostearyl alcohol | 1.0 |
| Lipophilic monostearic stearate | 1.0 |
| Squalane | 0.5 |
| Liposome dispersion of Example 4 | 5.0 |
| Polysorbate-60 | 1.5 |
| Sorbitan sesquioleate | 0.5 |
| Glycerin | 5.0 |
| Triethanolamine | 0.5 |
| Carboxyvinyl polymer | 0.2 |
| Preservative | q. s. |
| Pigments | q.s. |
| Perfume | q.s. |
| Distilled water | To 100 |

### <Formulation 3> Cream

**[Table 5]**

| Materials | Composition (% by weight) |
|---|---|
| Beeswax | 1.0 |
| Glyceryl stearate | 3.0 |
| Cetostearate | 2.5 |
| Polysorbate-60 | 1.0 |
| Sorbitan sesquioleate | 0.5 |
| Cetyl ethyl hexanoate | 0.5 |
| Squalane | 2.0 |
| Liquid paraffin | 5.0 |
| Mixture of active ingredients | 5.0 |
| Glycerin | 3.0 |
| Propylene glycol | 3.0 |
| Liposome dispersion of Example 5 | 5.0 |
| Preservative | q.s. |
| Pigments | q.s. |
| Perfume | q.s. |
| Distilled water | To 100 |

### <Formulation 4> Lotion

**[Table 6]**

| Materials | Composition (% by weight) |
|---|---|
| Glyceryl stearate | 1.5 |
| Polysorbate | 1.5 |
| Sorbitan sesquioleate | 0.5 |
| Cetyl ethyl hexanoate | 2.0 |
| Squalane | 2.0 |
| Lanolin | 2.0 |
| Glycerin | 3.0 |
| Carboxyvinyl polymer | 0.5 |
| Collagen hydrolysate | 1.0 |
| Triethanolamine | 0.5 |
| Liposome dispersion of Example 5 | 5.0 |
| Preservative | q.s. |
| Pigments | q.s. |
| Perfume | q. s. |
| Distilled water | To 100 |

As described above, the present submicron-liposome containing triterpenoid at high concentration has reliable safety onto the skin because toxic solvent is not employed, and has good chemical and colloidal stability because the submicron- liposome can be prepared in uniform size by self-assembling process. Further, because insoluble triterpenoid is loaded within liposome at a high concentration, the present liposome can exhibit much higher biological activity than free triterpenoid itself, and therefore, can be incorporated into cosmetic and pharmaceutical applications as an active ingredient.

Although preferred embodiments of the present invention have been described in detail hereinabove, it should be clearly understood that many variations or modifications may be achieved within the basic inventive concepts of the present invention.

## Claims

1. A method for preparing triterpenoid-containing liposomes, which comprises the following steps of:
(a) dispersing a triterpenoid having an acid moiety in a polyol while heating up to 60~70°C to prepare a dispersion;
(b) adding a base into the dispersion of step (a) converting the acid group in the triterpenoid to a salt to prepare a low viscosity dispersion;
(c) dissolving a phospholipid in ethanol of a room temperature to prepare an ethanol solution of phospholipid;
(d) adding the ethanol solution of step (c) into the dispersion of step (b) to prepare a mixture;
(e) adding the mixture of step (d) into distilled water and then emulsifying to prepare an emulsion; and
(f) adding an acid to the emulsion of step (e) to convert the triterpenoid salt of step (b) back to the acid form thereby preparing liposomes having diameters in a range of 0.001~10 µm and containing the triterpenoid in a range of 0.001~5% by weight based on the total weight of the liposome.

2. The method according to Claim 1, wherein said polyol of step (a) is selected from the group consisting of pentylene glycol, butylene glycol and propylene glycol.

3. The method according to Claim 1, wherein said base of step (b) is selected from the group consisting of triethanolamine, triisopropanolamine, potassium hydroxide, 2-aminobutanol, sodium hydroxide, ammonium hydroxide and calcium hydroxide.

4. The method according to Claim 3, wherein said base is the same normality as that of the triterpenoid of step (a) and added in an amount of 0.001~0.5% by weight based on the total weight of the liposome.

5. The method according to Claim 3, wherein said base is added in an amount to maintain pH of the dispersion of step (b) to a range of 10~11.

6. The method according to Claim 1, wherein said acid of step (f) is selected from the group consisting of adipic acid, boric acid, citric acid, acetic acid, formic acid, fumaric acid, lactic acid, glycolic acid, succinic acid, propionic acid, pyruvic acid and phosphoric acid.

7. The method according to Claim 6, wherein said acid is the same normality as that of the base of step (b).

8. The method according to Claim 6, wherein said acid is added in an amount to maintain pH of the liposome of step (f) in a range of 5~8.

9. The method according to Claim 1, wherein said phospholipid of step (c) has 0~3 of double bonds.

10. The method according to Claim 1, wherein said phospholipid of step (c) contains 70~95wt% of phosphatidylcholine.

11. Triterpenoid liposome prepared by the method according to Claim 1, wherein the content of said triterpenoid in the triterpenoid liposomes is in a range of 0.5~2.5% by weight based on the total weight of the liposome.

12. Triterpenoid liposome according to Claim 11, wherein is the diameter of the liposome is in a range of 0.1~0.2 µm.

13. Triterpenoid liposome according to Claim 11, wherein said triterpenoid is selected from the group consisting of ursolic acid, oleanolic, centella asiatica extract, betulinic acid, β-boswellic acid and their admixture.

14. Triterpenoid liposome according to Claim 11, wherein the content of said phospholipid in triterpenoid liposome is in a range of 0.001~15% by weight based on the total weight of liposome.

15. A skin-care composition containing triterpenoid liposome according to Claim 11.

16. The skin~care composition according to claim 15, wherein the composition has a formulation of skin softener, toilet water, nutrition toilet water, nutrition cream, massage cream, essence, eye cream, eye essence, cleansing cream, cleansing foam, cleansing water, pack, powder, body lotion, body oil, body essence, make-up base, foundation, hairdyes, shampoo, body cleaner, tooth paste, oral cleaner, patch or sprays.

## Patentansprüche

1. Verfahren zur Herstellung von triterpenoidhaltigen Liposomen, das die folgenden Stufen umfasst:
(a) Dispergieren eines Triterpenoids mit einer Säureeinheit in einem Polyol unter Erhitzen auf 60-70 °C, um eine Dispersion herzustellen;
(b) Einbringen einer Base in die Dispersion von Stufe (a), wobei die Säuregruppe in dem Triterpenoid in ein Salz umgewandelt wird, um eine Dispersion niedriger Viskosität herzustellen;
(c) Lösen eines Phospholipids in Ethanol mit Raumtemperatur, um eine Ethanollösung des Phospholipids herzustellen;
(d) Einbringen der Ethanollösung von Stufe (c) in die Dispersion von Stufe (b), um ein Gemisch herzustellen;
(e) Einbringen des Gemischs von Stufe (d) in destilliertes Wasser und nachfolgendes Emulgieren, wobei eine Emulsion hergestellt wird; und
(f) Zugabe einer Säure zu der Emulsion von Stufe (e), um das Triterpenoidsalz von Stufe (b) zurück in die Säureform umzuwandeln, wodurch Liposomen hergestellt werden, die Durchmesser im Bereich von 0,001-10 µm aufweisen und das Triterpenoid in einem Bereich von 0,001-5 Gew.-%, bezogen auf das Liposomgesamtgewicht, enthalten.

2. Verfahren nach Anspruch 1, wobei das Polyol von Stufe (a) aus der Gruppe von Pentylenglykol, Butylenglykol und Propylenglykol ausgewählt ist.

3. Verfahren nach Anspruch 1, wobei die Base von Stufe (b) aus der Gruppe von Triethanolamin, Triisopropanolamin, Kaliumhydroxid, 2-Aminobutanol, Natriumhydroxid, Ammoniumhydroxid und Calciumhydroxid ausgewählt ist.

4. Verfahren nach Anspruch 3, wobei die Base die gleiche Normalität wie das Triterpenoid von Stufe (a) aufweist und in einer Menge von 0,001-0,5 Gew.-%, bezogen auf das Liposomgesamtgewicht, zugegeben wird.

5. Verfahren nach Anspruch 3, wobei die Base in einer derartigen Menge zugegeben wird, dass der pH-Wert der Dispersion von Stufe (b) im Bereich von 10-11 gehalten wird.

6. Verfahren nach Anspruch 1, wobei die Säure von Stufe (f) aus der Gruppe von Adipinsäure, Borsäure, Citronensäure, Essigsäure, Ameisensäure, Fumarsäure, Milchsäure, Glykolsäure, Bernsteinsäure, Propionsäure, Brenztraubensäure und Phosphorsäure ausgewählt ist.

7. Verfahren nach Anspruch 6, wobei die Säure die gleiche Normalität wie die Base von Stufe (b) aufweist.

8. Verfahren nach Anspruch 6, wobei die Säure in einer derartigen Menge zugegeben wird, dass der pH-Wert der Liposomen von Stufe (f) im Bereich von 5-8 gehalten wird.

9. Verfahren nach Anspruch 1, wobei das Phospholipid von Stufe (c) 0-3 Doppelbindungen aufweist.

10. Verfahren nach Anspruch 1, wobei das Phospholipid von Stufe (c) 70-95 Gew.-% an Phosphatidylcholin enthält.

11. Triterpenoidliposom, das durch das Verfahren nach Anspruch 1 hergestellt ist, wobei der Gehalt an dem Triterpenoid in den Triterpenoidliposomen im Bereich von 0,5-2,5 Gew.-%, bezogen auf das Liposomgesamtgewicht, liegt.

12. Triterpenoidliposom nach Anspruch 11, wobei der Durchmesser des Liposoms im Bereich von 0,1-0,2 µm liegt.

13. Triterpenoidliposom nach Anspruch 11, wobei das Triterpenoid aus der Gruppe von Ursolsäure, Oleanolsäure, Centella-asiatica-Extrakt, Betulinsäure, β-Boswelliasäure und deren Gemischen ausgewählt ist.

14. Triterpenoidliposom nach Anspruch 11, wobei der Gehalt an dem Phospholipid in dem Triterpenoidliposom im Bereich von 0,001-15 Gew.-%, bezogen auf das Liposomgesamtgewicht, liegt.

15. Hautpflegezusammensetzung, die Triterpenoidliposomen nach Anspruch 11 enthält.

16. Hautpflegezusammensetzung nach Anspruch 15, wobei die Zusammensetzung die Formulierung eines Haut-Weichmachers, eines kosmetischen Wassers, eines nährenden kosmetischen Wassers, einer Nährcreme, einer Massagecreme, eines Konzentrats, einer Augencreme, eines Augenkonzentrats, einer Reinigungscreme, eines Reinigungsschaums, eines Reinigungswassers, einer Packung, eines Puders, einer Körperlotion, eines Körperöls, eines Körperkonzentrats, einer Makeupgrundlage, einer Grundierung, von Haarfarbstoffen, eines Shampoos, eines Körperreinigungsmittels, einer Zahnpasta, eines Mundreinigungsmittels, eines Pflasters oder von Sprays aufweist.

## Revendications

1. Procédé pour préparer des liposomes contenant un triterpénoïde, qui comprend les étapes suivantes consistant :
(a) à disperser un triterpénoïde ayant un groupe acide dans un polyol tout en chauffant jusqu'à 60~70 °C pour préparer une dispersion ;
(b) à ajouter une base dans la dispersion de l'étape (a), convertissant le groupe acide du triterpénoïde en un sel pour préparer une dispersion de faible viscosité ;
(c) à dissoudre un phospholipide dans de l'éthanol à température ambiante pour préparer une solution éthanolique du phospholipide ;
(d) à ajouter la solution éthanolique de l'étape (c) dans la dispersion de l'étape (b) pour préparer un mélange ;
(e) à ajouter le mélange de l'étape (d) dans de l'eau distillée puis à émulsifier pour préparer une émulsion ; et
(f) à ajouter un acide à l'émulsion de l'étape (e) pour reconvertir le sel du triterpénoïde de l'étape (b) sous la forme acide, préparant ainsi des liposomes ayant des diamètres dans une gamme de 0,001~10 µm et contenant le triterpénoïde dans une gamme de 0,001~5 % en poids par rapport au poids total du liposome.

2. Procédé selon la revendication 1, dans lequel ledit polyol de l'étape (a) est choisi dans le groupe constitué par le pentylèneglycol, le butylèneglycol et le propylèneglycol.

3. Procédé selon la revendication 1, dans lequel ladite base de l'étape (b) est choisie dans le groupe constitué par la triéthanolamine, la triisopropanolamine, l'hydroxyde de potassium, le 2-aminobutanol, l'hydroxyde de sodium, l'hydroxyde d'ammonium et l'hydroxyde de calcium.

4. Procédé selon la revendication 3, dans lequel ladite base a la même normalité que celle du triterpénoïde de l'étape (a) et est ajoutée en une quantité de 0,001~0,5 % en poids par rapport au poids total du liposome.

5. Procédé selon la revendication 3, dans lequel ladite base est ajoutée en une quantité pour maintenir le pH de la dispersion de l'étape (b) dans une gamme de 10~11.

6. Procédé selon la revendication 1, dans lequel ledit acide de l'étape (f) est choisi dans le groupe constitué par l'acide adipique, l'acide borique, l'acide citrique, l'acide acétique, l'acide formique, l'acide fumarique, l'acide lactique, l'acide glycolique, l'acide succinique, l'acide propionique, l'acide pyruvique et l'acide phosphorique.

7. Procédé selon la revendication 6, dans lequel ledit acide a la même normalité que celle de la base de l'étape (b).

8. Procédé selon la revendication 6, dans lequel ledit acide est ajouté en une quantité pour maintenir le pH du liposome de l'étape (f) dans une gamme de 5-8.

9. Procédé selon la revendication 1, dans lequel ledit phospholipide de l'étape (c) a 0~3 liaisons doubles.

10. Procédé selon la revendication 1, dans lequel ledit phospholipide de l'étape (c) contient 70~95 % en poids de phosphatidylcholine.

11. Liposome à triterpénoïde préparé par le procédé selon la revendication 1, dans lequel la teneur en ledit triterpénoïde dans les liposomes à triterpénoïde est dans une gamme de 0,5~2,5 % en poids par rapport au poids total du liposome.

12. Liposome à triterpénoïde selon la revendication 11, dans lequel le diamètre du liposome est dans une gamme de 0,1~0,2 µm.

13. Liposome à triterpénoïde selon la revendication 11, dans lequel ledit triterpénoïde est choisi dans le groupe constitué par l'acide ursolique, l'acide oléanolique, un extrait de *Centella asiatica,* l'acide bétulinique, l'acide β-boswellique et leur mélange.

14. Liposome à triterpénoïde selon la revendication 11, dans lequel la teneur en ledit phospholipide dans le liposome à triterpénoïde est dans une gamme de 0,001~15 % en poids par rapport au poids total de liposome.

15. Composition pour le soin de la peau, contenant un liposome à triterpénoïde selon la revendication 11.

16. Composition pour le soin de la peau selon la revendication 15, laquelle composition a une formulation d'émollient pour la peau, d'eau de toilette, d'eau de toilette nutritive, de crème nutritive, de crème de massage, d'essence, de crème pour les yeux, d'essence pour les yeux, de crème démaquillante, de mousse démaquillante, d'eau démaquillante, de pack, de poudre, de lotion corporelle, d'huile corporelle, d'essence corporelle, de base de maquillage, de fond de teint, de colorants capillaires, de shampoing, de nettoyant pour le corps, de pâte dentifrice, de nettoyant buccal, de timbre ou de pulvérisations.
